# EUROPÄISCHE PATENTSCHRIFT

(11) **EP 0 836 437 B1**
(45) Veröffentlichungstag und Bekanntmachung des Hinweises auf die Patenterteilung: **21.04.1999**
(21) Anmeldenummer: 95925810.4
(22) Anmeldetag: 03.07.1995
(51) Int. Cl.: A61B 17/80

(54) **VORRICHTUNG ZUR FIXATION VON KNOCHENFRAGMENTEN**
BONE FRAGMENT-FIXING DEVICE
DISPOSITIF DE FIXATION DE FRAGMENTS OSSEUX

(43) Veröffentlichungstag der Anmeldung: 22.04.1998
(73) Patentinhaber: Synthes AG Chur, 7002 Chur (CH)
(72) Erfinder: TALOS, Gilbert, CH-4515 Oberdorf (CH); JOOS, Ulrich, D-48147 Münster (DE)
(74) Vertreter: Lusuardi, Werther Giovanni, Dr.
(86) Internationale Anmeldenummer: EP9502567
(87) Internationale Veröffentlichungsnummer: WO9701991

(56) Entgegenhaltungen:
- EP-A- 0 506 420
- EP-A- 0 507 162
- FR-A- 2 556 583
- US-A- 2 406 832

## Beschreibung

Die Erfindung bezieht sich auf eine Vorrichtung zur Fixation von Knochenfragmenten gemäss dem Oberbegriff des Patentanspruchs 1.

Die Vorrichtung eignet sich insbesondere für orthognatische Korrekturen am Unterkiefer. Zur Behandlung von Fehlbildungen im Kiefer-Gesichtsbereich werden Osteotomien mit anschliessender Verlagerung der Knochenfragmente durchgeführt. Die Fixation der so gewonnenen knöchernen Fragmente gestaltet sich zum Teil schwierig, da nach Osteotomien mit Verlagerung der Fragmente keine kongruenten Knochenflächen vorhanden sind. Bei Unterkiefer Osteotomien wird die Situation noch zusätzlich erschwert, da im proximalen Fragment das Kiefergelenk lokalisiert ist, dessen Position nicht verändert werden sollte. Diese Umstände erschweren die Durchführung einer passgenauen funktionsstabilen Osteosynthese, die seit Jahren immer mehr angestrebt wird.

Die bisher bekannten Vorrichtungen auf diesem Gebiet, z.B. gemäss der DE-C 23.40.880, weisen alle den Nachteil auf, dass sie intraoperativ zu wenig anpassungsfähig sind und postoperativ praktisch keine Biegbarkeit aus der Plattenfläche heraus aufweisen.

Aus der EP-A- 0 507 162 ist weiterhin eine Vorrichtung zur Fixation von Knochenfragmenten bekannt mit einer longitudinalen Knochenplatte, die an ihren beiden Enden mindestens je ein durchgehendes Schraubenloch aufweist. Die bekannte Knochenplatte weist ferner ein zentrales, sich in Richtung der Plattenlängsachse erstreckendes Langloch auf. Schliesslich ist auch ein Gleiter vorgesehen, der auf den das Längsloch seitlich begrenzenden Stegen in Richtung der Plattenlängsachse verschiebbar angeordnet ist. Damit ist allerdings nur eine beschränkte Justierung der Knochenfragmente zueinander möglich.

Hier will die Erfindung Abhilfe schaffen. Der Erfindung liegt die Aufgabe zugrunde, eine Vorrichtung zur Fixation von Knochenfragmenten zu schaffen, die einerseits eine intraoperative Anpassbarkeit bei der Justierung der Knochenfragmente zueinander gestattet und anderseits eine gewisse Flexibilität der Vorrichtung quer zur Plattenfläche ermöglicht.

Die Erfindung löst die gestellte Aufgabe mit einer Vorrichtung, welche die Merkmale des Anspruchs 1 aufweist, sowie einer Knochenplatte für diese Vorrichtung, welche die Merkmale des Anspruchs 6 aufweist.

Weitere vorteilhafte Ausgestaltungen der Erfindung sind in den abhängigen Ansprüchen gekennzeichnet.

Die durch die Erfindung erreichten Vorteile sind im wesentlichen darin zu sehen, dass dank der erfindungsgemässen Vorrichtung eine grosse Flexibilität bei der Justierung der Knochenfragmente zueinander, insbesondere der Okklusion bei einer maxillofazialen Anwendung, erzielt wird. Durch den auf die longitudinale Knochenplatte aufgesetzten Gleiter mit quer zur Plattenlängsachse verlaufendem Langloch, wird eine begrenzte 2-dimensionale Einstellung in der x/y-Ebene der Knochenplatte ermöglicht. Durch die zusätzliche (konstruktiv durch das Langloch bedingte) Flexibilität der longitudinale Knochenplatte in z-Richtung, d.h. aus der x/y-Ebene heraus, wird eine weitere Anpassung bei einer nach der orthognatischen Korrektur entstandenen Fehlstellung des Kiefergelenkes ermöglicht.

Die Erfindung und Weiterbildungen der Erfindung werden im folgenden anhand eines Ausführungsbeispiels noch näher erläutert.

Es zeigen:
Fig. 1 eine perspektivische Ansicht der auf einem Unterkiefer montierten erfindungsgemässen Vorrichtung;
Fig. 2 eine Aufsicht auf die Knochenplatte der erfindungsgemässen Vorrichtung;
Fig. 3 einen Längsschnitt durch die Knochenplatte nach Fig. 2 längs der Linie III-III;
Fig. 4 eine Aufsicht auf den Gleiter der erfindungsgemässen Vorrichtung;
Fig. 5 einen Längsschnitt durch den Gleiter nach Fig. 4 längs der Linie V-V;
Fig. 6 einen Längsschnitt durch den Gleiter nach Fig. 4 längs der Linie VI-VI;
Fig. 7 eine perspektivische Ansicht der auf einem Unterkiefer montierten erfindungsgemässen Vorrichtung mit intraoperativer Anpassungsmöglichkeit in der Längsrichtung der Knochenplatte;
Fig. 8 eine perspektivische Ansicht der auf einem Unterkiefer montierten erfindungsgemässen Vorrichtung mit intraoperativer Anpassungsmöglichkeit quer zur Knochenplatte;
Fig. 9 eine perspektivische Ansicht der auf einem Unterkiefer montierten Knochenplatte der erfindungsgemässen Vorrichtung im postoperativen Endzustand mit grösstmöglicher Anpassungsmöglichkeit senkrecht zu Ebene der Knochenplatte; und
Fig. 10 eine perspektivische Ansicht der auf einem Unterkiefer montierten erfindungsgemässen Vorrichtung im postoperativen Endzustand mit minimaler Anpassungsmöglichkeit senkrecht zu Ebene der Knochenplatte.

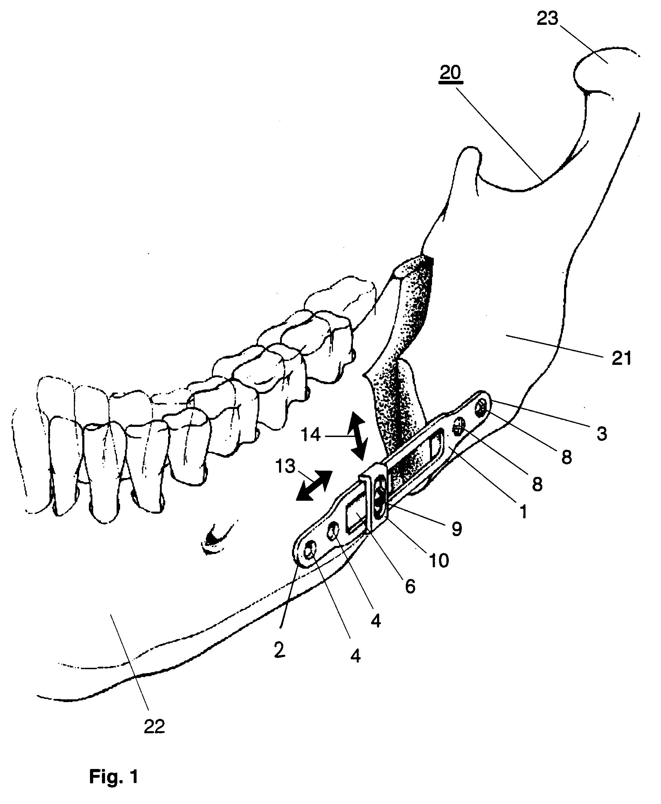

Die in Fig. 1 dargestellte erfindungsgemässe Vorrichtung besteht im wesentlichen aus einer longitudinalen Knochenplatte 1 und einem darauf verschieblich angeordneten Gleiter 10.

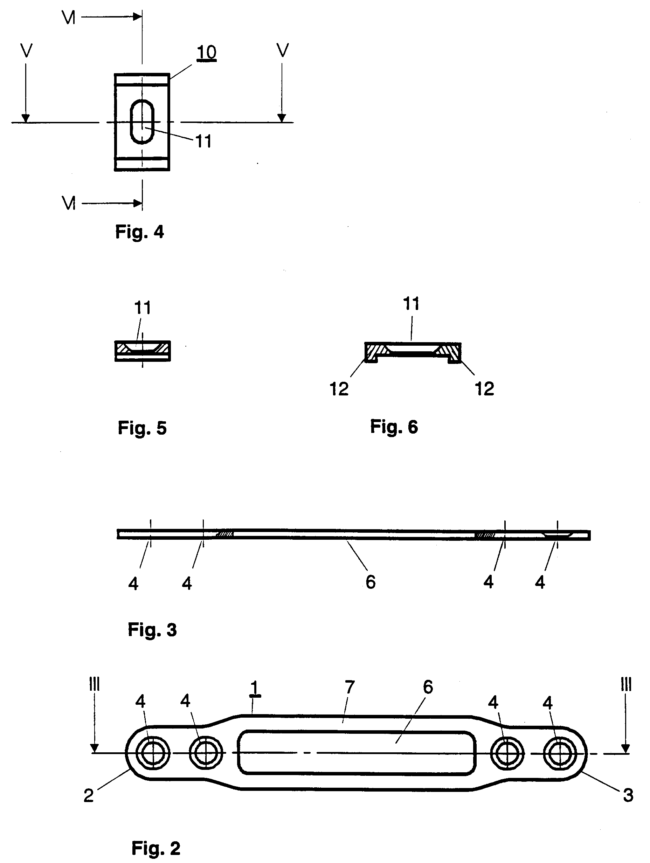

Die in den Fig. 2 und 3 im Detail dargestellte longitudinale Knochenplatte 1 weist an ihren beiden Enden 2,3 je zwei durchgehende Schraubenlöcher 4 auf, durch welche Kortikalisschrauben 8 (Fig. 1) in den Unterkieferknochen 20 gedreht werden können. Die Knochenplatte 1 weist ferner ein zentrales, sich in Richtung der Plattenlängsachse 5 ersteckendes Langloch 6 auf. Bei einer Gesamtlänge der Knochenplatte 1 von 30 - 50 mm (typischerweise 44 mm), einer im Zentrum der Knochenplatte 1 gemessenen Maximalbreite von 6 - 8 mm (z.B. 7 mm) und einer Dicke von 0,5 - 0,8 mm (z.B. 0,7 mm) wird die Länge des Längsloches 6 zweckmässigerweise im Bereich von 18 - 27 mm gewählt und beträgt typischerweise 22,8 mm; die Breite des Langloches 6 wird zweckmässigerweise im Bereich von 3 - 5 mm gewählt und beträgt typischerweise 4 mm.

Der in den Fig. 4 - 6 im Detail dargestellte Gleiter 10 ist im wesentlichen ebenfalls plattenförmig ausgebildet und weist ein Langloch 11 auf. Die endständigen Schienen 12 sind derart am Gleiter 10 angeordnet und dimensioniert, dass der Gleiter 10 auf den das Langloch 6 der Knochenplatte 1 seitlich begrenzenden Stegen 7 aufgesetzt werden kann und in Richtung der Plattenlängsachse 5 verschoben werden kann. Das Langloch 11 des Gleiters 10 verläuft dabei, im aufgesetzten Zustand, quer zur Plattenlängsachse 5. Bei einer Länge des Gleiters 10 von 9 mm wird die Länge des Langloches 11 zweckmässigerweise im Bereich von 3 - 5 mm gewählt und beträgt typischerweise 4 mm, d.h. entsprechend der Breite des Langlochs 6 der Knochenplatte 1.

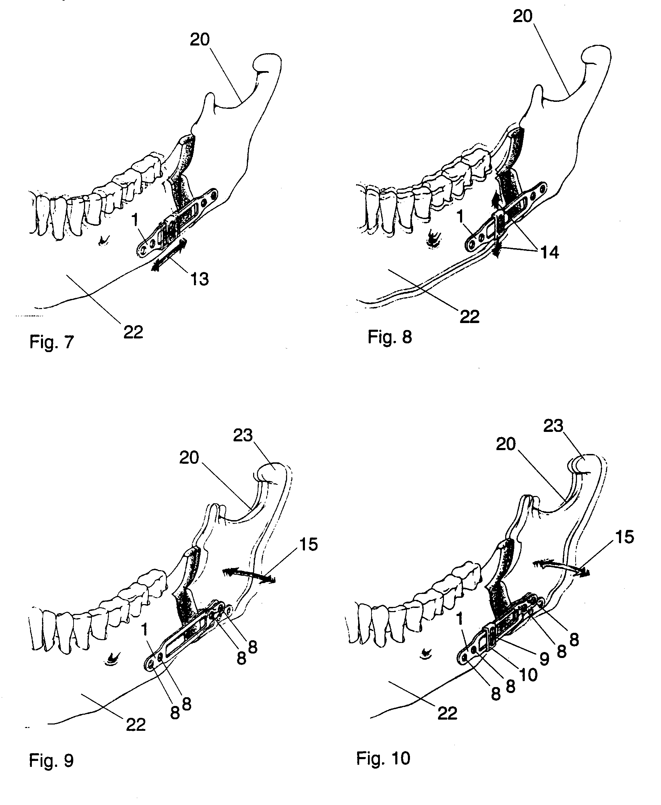

Anhand der Fig. 1 sowie der Fig. 7 - 10 wird nun die Anwendung der erfindungsgemässen Vorrichtung zur Fixation der Kieferfragmente während und nach einer Umstellungs-Osteotomie (Dysgnathie) am Unterkiefer beschrieben. Dazu wird intraoperativ nach einer sagittalen Split-Osteotomie am Knochenfragment 21, die Knochenplatte 1 an ihrem einen Ende 2 durch die Bohrlöcher 4 mit zwei Kortikalisschrauben 8 fixiert (monokortikale Verschraubung um eine Gelenkrotation zu verhindern). Temporar wird der Gleiter 10 auf der Knochenplatte 1 im Bereich des Langlochs 6 am Knochenfragment 22 mittels der Schraube 9 - ebenfalls monokortikal - befestigt. dieser Zustand ist in den Fig. 7 und 8 dargestellt.

Eine weitere solche erfindungsgemässe Vorrichtung 1,10 wird auch auf der anderen - in der Fig. 1 nicht sichbaren - Seite des Unterkiefers 20 befestigt.

Danach wird die intramaxilläre Immobilisation gelöst und der Unterkiefer manuell in die zentrale Gelenkposition geführt. Ist die Fixation in korrekter Weise erfolgt, lässt sich der Unterkiefer in die präoperativ geplante Okklusion führen. Ist dies nicht der Fall, kann entweder das Gelenk leicht luxiert sein, was sich durch einen offenen Biss bemerkbar macht , oder aber die sagittale Einstellung ist nicht korrekt, was einen singulären Antagonismus hervorrufen würde. Ist einer dieser Fälle aufgetreten, wird die Schraube 9 im Langloch 11 des Gleiters 10 gelockert und der Unterkiefer entsprechend manuell in die richtige Position eingestellt und die Schraube 9 erneut fixiert und das Vorgehen gegebenenfalls wiederholt.

Durch das in Längsrichtung der Knochenplatte 1 angeordnete Langloch 6 und das quer dazu angeordnete Langloch 11 des Gleiters 10 wird eine drei-dimensionale Flexibilität der erfindungsgemässen Vorrichtung 1,10 bei der Einstellung der Kieferposition gewährleistet. Diese Flexibilität besteht sowohl in der durch den Pfeil 13 angedeuteten x-Richtung (Fig. 7), als auch in der durch den Pfeil 14 (Fig. 8) angedeuteten y-Richtung.

Nach dem Einstellen der genauen gegenseitigen Position (Okklusion) von Unter- und Oberkiefer, wird die Schraube 9 im Langloch 11 des Gleiters 10 fest angezogen, so dass der Gleiter 10 in x-Richtung und y-Richtung sowohl gegenüber der Knochenplatte 1 als auch gegenüber dem Knochenfragment 22 blockiert ist.
Daraufhin werden - wie in Fig. 9 gezeigt - zwei weitere Kortikalisschrauben 8 durch die noch freien Schraubenlöcher 4 am anderen Ende 2 der Knochenplatte 1 geführt und im Knochenfragment 22 fixiert und der Gleiter 10 entfernt.

Durch die gegenüber üblichen Knochenplatten reduzierte Dicke und relativ grosse Breite der Knochenplatte 1 wird eine Anpassung in der Richtung der z-Achse (Pfeil 15) und somit eine begrenzte Korrektur von Achsenfehlern des Kiefergelenkes 23 ohne Verlust der Steifigkeit in der y-Achse ermöglicht.

Wie in Fig. 9 dargestellt ergibt sich postoperativ eine maximale Anpassungsmöglichkeit in Richtung der z-Achse (Pfeil 15), wenn der Gleiter 10 ganz weggelassen wird. Wird wie in Fig. 10 der Gleiter 10 montiert, so ergibt sich postoperativ eine minimale Anpassungsmöglichkeit in Richtung der z-Achse (Pfeil 15).

## Patentansprüche

1. Vorrichtung zur Fixation von Knochenfragmenten mit
A) einer longitudinalen Knochenplatte (1), die an ihren beiden Enden (2,3) mindestens je ein durchgehendes Schraubenloch (4) sowie ein zentrales, sich in Richtung der Plattenlängsachse (5) erstreckendes Langloch (6) aufweist; und mit
B) einem Gleiter (10), der auf den, das Langloch (6) seitlich begrenzenden Stegen (7) in Richtung der Plattenlängsachse (5) verschiebbar angeordnet ist, dadurch gekennzeichnet, dass
C) der Gleiter (10) ein, im aufgesetzten Zustand, quer zur Plattenlängsachse (5) verlaufendes Langloch (11) aufweist; und
D) die Länge des Langlochs (6) der Knochenplatte (1) 45 - 60 % der Gesamtlänge der Knochenplatte (1) beträgt.

2. Vorrichtung nach Anspruch 1, dadurch gekennzeichnet, dass die Länge des Langlochs (6) 50 - 54 % der Gesamtlänge der Knochenplatte (1) beträgt.

3. Vorrichtung nach Anspruch 1 oder 2, dadurch gekennzeichnet, dass die Breite des Langlochs (6) 50 - 65 %, vorzugsweise 55 - 59 % der Gesamtbreite der Knochenplatte (1) beträgt.

4. Vorrichtung nach einem der Ansprüche 1 - 3, dadurch gekennzeichnet, dass die Dicke der Knochenplatte (1) 8 - 12 %, vorzugsweise 9 - 11 % der Gesamtbreite der Knochenplatte (1) beträgt.

5. Vorrichtung nach einem der Ansprüche 1 - 4, dadurch gekennzeichnet, dass die Länge des Langlochs (11) des Gleiters (10) im wesentlichen der Breite des Langlochs (6) der Knochenplatte (1) entspricht.

6. Knochenplatte (1) für eine Vorrichtung nach Anspruch 1, dadurch gekennzeichnet, dass die Länge des Langlochs (6) 45 - 60 %, vorzugsweise 50 - 54 % der Gesamtlänge der Knochenplatte (1) beträgt.

7. Knochenplatte nach Anspruch 6, dadurch gekennzeichnet, dass die Breite des Langlochs (6) 50 - 65 %, vorzugsweise 55 - 59 % der Gesamtbreite der Knochenplatte (1) beträgt.

8. Knochenplatte nach anspruch 6 oder 7, dadurch gekennzeichnet, dass die Dicke der Knochenplatte (1) 8 - 12 %, vorzugsweise 9 - 11 % der Gesamtbreite der Knochenplatte (1) beträgt.

## Claims

1. Device for the fixation of bone fragments with
A) a longitudinal bone plate (1) which is provided at both of its ends (2,3) each with at least one through-hole (4) for screws as well as a central longitudinal hole (6) extending in the direction of the longitudinal axis (5) of the bone plate (1); and with
B) a slide (10) displaceably arranged on the webs (7) which laterally limit the longitudinal hole (6) and in the direction of the longitudinal axis (5) of the bone plate (1);
**characterized in that**
C) the slide (10) is provided with a longitudinal hole (11), extending transversely to the longitudinal axis (5) of the bone plate (1) when the slide (10) is mounted thereon; and
D) the length of the longitudinal hole (6) of the bone plate (1) amounts to 45-60% of the total length of the bone plate (1).

2. Device according to claim 1, characterized in that the length of the longitudinal hole (6) amounts to 50-54% of the total length of the bone plate (1).

3. Device according to claim 1 of 2, characterized in that the width of the longitudinal hole (6) amounts to 50-65%, preferably 55-59%, of the total width of the bone plate (1).

4. Device according to one of the claims 1-3, characterized in that the thickness of the bone plate (1) amounts to 8-12%, preferably 9-11%, of the total width of the bone plate (1).

5. Device according to one of the claims 1-4, characterized in that the length of the longitudinal hole (11) of the slide (10) essentially corresponds to the width of the longitudinal hole (6) of the bone plate (1).

6. Bone plate (1) for a device according to claim 1, characterized in that the length of the longitudinal hole (6) amounts to 45-60%, preferably 50-54%, of the total length of the bone plate (1).

7. Bone plate according to claim 6, characterized in that the width of the longitudinal hole (6) amounts to 50-65%, preferably to 55-59%, of the total width of the bone plate (1).

8. Bone plate according to claim 6 or 7, characterized in that the thickness of the bone plate (1) amounts to 8-12%, preferably to 9-11%, of the total width of the bone plate (1).

## Revendications

1. Dispositif pour la fixation de fragments d'os, comportant :
A) une plaque longitudinale pour os (1) qui est traversée à chacune de ses deux extrémités (2, 3) par au moins un trou pour vis (4), et qui présente un trou oblong central (6) qui s'étend dans la direction de l'axe longitudinal (5) de la plaque, ainsi que
B) un coulisseau (10) qui est disposé de manière à pouvoir coulisser dans la direction de l'axe longitudinal (5) de la plaque sur les ailes (7) délimitant latéralement le trou oblong (6),
caractérisé en ce que
C) le coulisseau (10) présente un trou oblong (11) qui, lorsqu'il est en position de pose, s'étend transversalement par rapport à l'axe longitudinal (5) de la plaque; et
D) la longueur du trou oblong (6) de la plaque pour os (1) vaut de 45 à 60% de la longueur totale de la plaque pour os (1).

2. Dispositif selon la revendication 1, caractérisé en ce que la longueur du trou oblong (6) vaut de 50 à 54% de la longueur totale de la plaque pour os (1).

3. Dispositif selon les revendications 1 ou 2, caractérisé en ce que la largeur du trou oblong (6) vaut de 50 à 65%, de préférence de 55 à 59%, de la largeur totale de la plaque pour os (1).

4. Dispositif selon l'une des revendications 1 à 3, caractérisé en ce que l'épaisseur de la plaque pour os (1) vaut de 8 à 12%, de préférence de 9 à 11%, de la largeur totale de la plaque pour os (1).

5. Dispositif selon l'une des revendications 1 à 4, caractérisé en ce que la longueur du trou oblong (11) du coulisseau (10) correspond essentiellement à la largeur du trou oblong (6) de la plaque pour os (1).

6. Plaque pour os (1) pour un dispositif selon la revendication 1, caractérisée en ce que la longueur du trou oblong (6) vaut de 45 à 60%, de préférence de 50 à 54%, de la longueur totale de la plaque pour os (1).

7. Plaque pour os selon la revendication 6, caractérisée en ce que la largeur du trou oblong (6) vaut de 50 à 65%, de préférence de 55 à 59%, de la largeur totale de la plaque pour os (1).

8. Plaque pour os selon les revendications 6 ou 7, caractérisée en ce que l'épaisseur de la plaque pour os (1) vaut de 8 à 12%, de préférence de 9 à 11%, de la largeur totale de la plaque pour os (1).
